# EUROPEAN PATENT SPECIFICATION

(11) **EP 1 512 394 B1**
(45) Date of publication and mention of the grant of the patent: **26.03.2008**
(21) Application number: 03019532.5
(22) Date of filing: 01.09.2003
(51) Int. Cl.: A61K 9/20, A61K 47/36, A61K 31/137, A61K 31/138, A61K 31/166, A61K 31/522

(54) **Universal controlled-release composition comprising chitosan**
Universelle Zusammensetzung zur kontrollierten Wirkstoffabgabe enthaltend Chitosan
Composition universelle à libération controlée comprenant du chitosane

(43) Date of publication of application: 09.03.2005
(73) Proprietor: JPM - The Jordanian Pharmaceutical Manufacturing Co. Ltd., 11710 Naor (JO)
(72) Inventor: Badwan, Adnan Ali, Amman 11185 (JO); Al-Remawi, Mayyas Mohammad Ahmad, 13713 Russeifa (JO); Salem, Mutaz Bellah A. W. Sheikh, 22110 Irbid (JO)
(74) Representative: Winkler, Andreas Fritz Ernst

(56) References cited:
- WO-A-00/04086
- US-A- 4 814 176
- DOS SANTOS NOGUEIRA C C ET AL: "Evaluation of new polysaccharides networks for extended-release purposes: Mesquite seed gum (MSG), xanthan gum and chitosan" REVISTA BRASILEIRA DE CIENCIAS FARMACEUTICAS/BRAZILIAN JOURNAL OF PHARMACEUTICAL SCIENCES 2003 BRAZIL, vol. 39, no. 3, 2003, pages 273-288, XP009026704 ISSN: 1516-9332
- MESHALI M M ET AL: "Effect of interpolymer complex formation of chitosan with pectin or acacia on the release behaviour of chlorpromazine hydrochloride" INTERNATIONAL JOURNAL OF PHARMACEUTICS (AMSTERDAM), vol. 89, no. 3, 1993, pages 177-181, XP009026688 ISSN: 0378-5173
- HASAN E I ET AL: "Assessment of a controlled release hydrophilic matrix formulation for metoclopramide HCl" EUROPEAN JOURNAL OF PHARMACEUTICS AND BIOPHARMACEUTICS, ELSEVIER SCIENCE PUBLISHERS B.V., AMSTERDAM, NL, vol. 55, no. 3, May 2003 (2003-05), pages 339-344, XP004425154 ISSN: 0939-6411
- DUMITRIU S ET AL: "Polyionic hydrogels as support for controlled release of nifedipine" 27TH INTERNATIONAL SYMPOSIUM ON CONTROLLED RELEASE OF BIOACTIVE MATERIALS AND 3RD CONSUMER AND DIVERSIFIED PRODUCTS CONFERENCE. PROCEEDINGS BOOK 2000. PARIS, FRANCE, JULY 7 - 13, 2000, DEERFIELD, IL: CRS, US, 7 July 2000 (2000-07-07), pages 455-456, XP002189752
- BABU G V MURALI MOHAN ET AL: "Controlled release of diclofenac sodium by gum karaya - chitosan complex coacervate: In vivo evaluation" INDIAN JOURNAL OF PHARMACEUTICAL SCIENCES, vol. 63, no. 5, September 2001 (2001-09), pages 408-412, XP001179916 ISSN: 0250-474X
- SINGLA A K ET AL: "CHITOSAN: SOME PHARMACEUTICAL AND BIOLOGICAL ASPECTS - AN UPDATE" JOURNAL OF PHARMACY AND PHARMACOLOGY, LONDON, GB, vol. 53, no. 8, August 2001 (2001-08), pages 1047-1067, XP008019188 ISSN: 0022-3573
- BHARDWAJ T R ET AL: "Natural gums and modified natural gums as sustained-release carriers." DRUG DEVELOPMENT AND INDUSTRIAL PHARMACY. UNITED STATES OCT 2000, vol. 26, no. 10, October 2000 (2000-10), pages 1025-1038, XP009026702 ISSN: 0363-9045

## Description

The present invention is directed to an universal controlled-release composition. The present invention is also directed to the preparation and use of said composition.

### Background of the invention

Over the past years, concepts of controlled drug delivery have been developed extensively, and controlled-release systems designed for this purpose are widely used today. Such systems generally comprise a pharmacologically inactive matrix including one or more active agents which are released from the matrix. Controlling drug delivery may therapeutically be desirable with respect to time (temporal control) and/or place (spatial control).

In case of temporal control, drug release may be sustained or otherwise modified. Alterations in the releasing rates may produce constant drug levels locally at the site of delivery and even constant plasma concentrations. This may result in significantly improved therapeutic effects, e.g. through greater efficacy and/or reduced toxicity. Furthermore, lowering the frequency of administration may lead to a more convenient administration regimen and thus to a better patient's compliance. With respect to temporal control, one focus lies on dermal systems, e.g. plaster. However, systems for oral administration, such as tablets and capsules, are also well known in the art.

Furthermore, oral systems may offer the opportunity to spatially control drug delivery within the gastrointestinal tract. This may be of great advantage, for example, if the intended therapeutic success depends on the portion of the gastrointestinal tract where the pharmacologically active agent should be delivered.

The use of hydrophilic polymers in composing an inactive matrix for controlled release of an active agent are known in the art. For controlled-release solid dosage forms comprising a drug dispersed uniformly in hydrophilic polymers, release of the drug is controlled primarily by diffusion of the drug, or by surface erosion of the hydrophilic polymers in the surrounding medium, or by a combination of the two processes.

In the development of controlled-release pharmaceutical compositions, several polymers, such as hydrophilic polysaccharides, e.g. xanthan gum, sodium alginate and chitosan, have been employed.

### 1. Xanthan gum

Xanthan gum, also known as corn sugar gum, is a high molecular weight polysaccharide being produced by *Xanthomonas campestris.* Because of its extraordinary resistance against enzymatic attacks, it is well suitable for oral controlled-release matrix preparations. In such compositions, it may serve as a vicolyzing agent, which, upon contact with gastrointestinal fluid, instantaneously viscolyses to trap the gas generated by a gas generating component (e.g. carbonates, bicarbonates, sulfites) (*cf*. U.S. patent No. 6,261,601).

Xanthan gum has been used alone or in combination with other polymers in controlled-release compositions. U.S. patent No. 4,309,405 used a combination of xanthan gum and hydroxypropylmethyl cellulose (HPMC), hydroxypropyl cellulose and ethyl cellulose in coated tablets for controlled release. These tablets contained 30-72% (by weight) of a mixture of polymers. The British patent No. 770114 used combinations of locust bean gum and xanthan gum in buccal tablets containing a phenothiazine compound for sustained release up to 2 hours. It is well established that xanthan gum not only retards *in vitro* drug release and provides time-independent release kinetics but can also work effectively *in vivo* and establishes constant drug plasma levels. It was found that the release of a water-soluble drug (chlor-pheniramine maleate) and a water-insoluble drug (theopylline) from tablets containing low concentrations of xanthan gum occurred mainly via diffusion and erosion, respectively. Drug release from tablets containing xanthan gum was slightly faster in an acidic environment due to more rapid initial surface erosion than at higher pH. After hydration of the gum, drug release was essentially pH-independent (1). The swelling of xanthan gum matrix tablets was reciprocal with salt concentration. Depending on the solubility of the product, the drug release from this matrix was regulated by its swelling behaviour (2). The release of an insoluble drug followed a direct relationship with swelling of the polymer matrix, while a reciprocal relationship was observed with soluble drugs. Swelling of xanthan gum showed a square root of time dependency whereas drug release was almost time-independent (2).

Talukdar *et al*. (3) made a comparison based on compaction and *in vitro* drug release between xanthan gum and HPMC as matrices for controlled release. They found that with respect to controlled drug release, xanthan gum matrices showed pharmaceutical as well as economical advantages. These included absence of initial burst release, higher drug retarding ability, more reproducibility in drug release, and the possibility of a zero order release kinetic. Regarding the influence of ionic strength of the medium, xanthan gum exhibited the disadvantage that the drug release was influenced by the total salt concentration within the gastrointestinal tract while drug release from HPMC matrices was independent of ionic strength. However, this ionic strength dependency should not be considered as a total failure of xanthan gum for controlling drug release. Compaction characteristics of xanthan gum were quite similar to HPMC whereas flowability of xanthan gum was better.

### 2. Chitosan

Chitosan is derived from chitin, a bountiful natural polysaccharide obtainable from crustacean shells. Partial deacetylation of chitin gives rise to chitosan, a linear polysaccharide with interspersed D-glucosamine and acetyl-D-glucosamine units.

The use of chitosan, alone or in combination, is also known in the art. Chitosan appeared suitable for controlled release oral drug delivery systems because of its physicochemical and pharmaceutical properties. First, chitosan direct compression tablets triggered the sustained release of the water-insoluble drug prednisolone (4). Second, chitosan is a polymer that has a good mucoadhesion property. Actually, mucoadhesive polymeric systems have generated significant interest over the years as a method to improve the performance of controlled relase drug delivery systems. This is because such systems can localise the formulation at the site of interest for local or systemic delivery, optimise contact with the gastrointestinal tissue surface to improve drug permeability, and they can be easily combined with auxiliary agents, such as enzyme inhibitors, adhesion and penetration enhancers. One interesting example is the use of chitosan microspheres for the specific delivery of amoxycillin to the gastric cavity to treat gastric diseases, such as peptic ulcer associated with *Helicobacter pylori* infections. This system is valuable due to the following advantages. Although there are many anti-microbials which are effective against *Helicobacter pylori in vitro,* their effect is limited *in vivo* due to low accessibility of the drug to the bacterial mucosal home sites. In case of chitosan microspheres, anti-microbials are much more effective because of the mucoadhesive properties through which they stuck to the stomach wall. The particles of chitosan have the ability to disperse in the stomach resulting in broader contact with the site of delivery. A valuable quality of chitosan is recognised anti-ulcer properties, which suggest its potential adjuvant effect in this particular application (5). Third, chitosan cross-linked with acetic anhydride and glutaraldehyde forms a spongy structure upon exposure to dissolution medium. This spongy structure was prepared as controlled release drug carrier to be used in tooth extraction, implantation and oral administration (6). Fourth, chitosan conjugates with either drugs or other chemical moieties to modify its properties are known. Chitosan-EDTA was developed as a novel bioadhesive polymer that protects peptide drugs from luminal degradation by aminopeptidase N (7). The conjugation of indomethacin with chitosan partially substituted with hydrophilic residues, triethylene glycol glutarate and triethylene glycol choline ether glutarate was described (8). Chitosan as a drug carrier is well documented in the literature (4, 9) as well as the release of drugs from chitosan matrix (9).

Chitosan beads can be formed by dropping chitosan acidic solution into a sodium tripolyphosphate solution and/or polyanionic polymer (e.g. alginate) solution. These beads were prepared as controlled release carriers. Insoluble hydrogels are formed upon the reaction of polyanionic polymers with chitosan (10-12).

A combination of chitosan and xanthan gum has also been described. U.S. Patent 5,620,706 disclosed the preparation of chitosan in combination with xanthan gum as an insoluble hydrogel. This system was used to immobilise biological materials. Also, the formed complex was found to be slowly dissociating in acidic medium and thus to provide for sustained release compounds in near neutral pH. U.S. Patent 4,808,707 disclosed the development of chitosan-alginate capsules. The permeability of the capsule walls was adjusted by substituting esterified alginic acid with a portion of the metal alginate normally used in fabrication.

U.S. Patent 4,814,176 discloses a sustained-release preparation comprising chitosan and anionic polymer compounds such as those having, for example, a carboxyl group. A broad range of pharmaceutically active agents is mentioned with propranolol and salbutamol amongst them.

WO 00/04086 discloses hydrogels based on chitosan and xanthan for controlled-release of sensitive substances such as vitamins, amino acids, nucleic acids and polypeptides (*cf.* e.g. the abstract).

Dos Santos Nogueira *et al.* (14) disclose extended-release hydrogels comprising mesquite seed gum (MSG) together with xanthan gum, and MSG together with chitosan, respectively. Propranolol and theophyllin were used as model drugs.

Meshali and Gabr (15) disclose interpolymer complexes of chitosan and pectin or chitosan and acacia and also their use for sustained-release of chlorpromazine.

Hasan *et al.* (16) disclose a controlled-release matrix of chitosan and sodium alginate using metoclopramide.

Dumitriu *et al.* (17) disclose a polyionic complex obtained by ionic cross-linking of xanthan with chitosan and their use in a controlled release system with nifedipine.

Murali Mohan Babu *et al.* (18) discloses microcapsules prepared from gum karaya and chitosan for controlled-release of diclofenac.

However, formulations for controlled-release compositions are generally developed each for an individual drug. Such a developing procedure involves plenty of time and money. Thus, it would be of great advantage to have a range of universal formulations being suited to provide for a controlled-release behaviour of most active agents.

Thus, there is still a need for the development of controlled-release compositions universally suitable for drugs belonging to only a few groups of a certain classification.

It is therefore an object of the present invention to provide for an universal pharmaceutical composition formulated for the controlled-release of a well defined group of active agents.

Unexpectedly, it turned out that drugs classified as "basic agents" showed similar controlled-release properties when a polysaccharide mixture of xanthan gum and chitosan was used as a matrix.

In particular, it is therefore an object of the present invention to provide for an universal pharmaceutical composition formulated for the controlled-release of basic active agents.

The controlled-release formulation provided by the present invention has several advantages over the prior art. In particular, the composition is easy and inexpensive to prepare and suited for direct compression. No organic solvents are contained in the composition and the components used are biocompatible. Additionally, the composition has less dose dumping properties.

### Summary of the invention

The present invention provides a controlled-release pharmaceutical composition comprising at least one basic active agent and an inactive matrix, said matrix comprising a mixture of hydrophilic polysaccharide polymers, said mixture consisting of chitosan and xanthan gum, wherein the ratio of xanthan gum and chitosan is about 1:1, and the ratio of the basic active agent and hydrophilic polysaccharide polymer mixture is in the range from about 1:1 to about 1:5.

In just another embodiment of the present invention, the ratio of the basic active agent and hydrophilic polysaccharide polymer mixture is about 1:3.

In a particularly preferred embodiment, the basic active agent is selected from the group comprising ambroxol, metoclopramide, propranolol, and salts and derivatives thereof.

In just another embodiment of the present invention, the controlled-release pharmaceutical composition optionally comprises one or more additional pharmaceutically acceptable fillers.

In a preferred embodiment of the present invention, the one or more pharmaceutically acceptable fillers are selected from the group comprising calcium hydrogen phosphate, mannitol, Avicel PH 101, sodium bicarbonate and the like.

In another embodiment of the present invention, the ratio of the basic active agent and the additional one or more pharmaceutically acceptable fillers on the one hand and hydrophilic polysaccharide mixture on the other hand is in the range of 1:1 to about 1:5.

In just another embodiment of the present invention, the ratio of the basic active agent and the additional one or more pharmaceutically acceptable fillers on the one hand and hydrophilic polysaccharide mixture on the other hand is about 1:3.

In a preferred embodiment of the present invention, the basic active agent is present in an amount from about 2.5% by weight to about 10% by weight, and the one or more pharmaceutically acceptable fillers are present in an amount from about 15% by weight to about 22.5% by weight.

In just another preferred embodiment of the present invention, the basic active agent is a basic active agent of low strength.

In a particularly preferred embodiment of the present invention, the basic active agent of low ionic strength is selected from the group comprising salbutamol and ketotifen and salts and derivatives thereof.

In a further aspect, the present invention is directed to a method for preparation of a controlled-release pharmaceutical composition according to the present invention, said method comprising the following steps:
(a) preparing a mixture of hydrophilic polysaccharide polymers consisting of chitosan and xanthan gum, wherein the ratio of xanthan gum and chitosan is about 1:1;
(b) mixing said mixture with a basic active agent, and optionally one or more additional pharmaceutically acceptable fillers;
(c) processing the preparation into granules;
(d) compacting the granules, preferably into a tablet.

In another aspect, the present invention is directed to a use of a controlled release pharmaceutical composition according to the invention for the manufacture of a medicament for the treatment of an animal, preferably a mammal, and most preferably man.

The term "controlled release" or "controlled delivery" as used in the context of the present invention refers to temporal and/or spatial control. "Temporal" can indicate a "sustained release" and "sustained delivery", respectively, or any release and delivery altered or modulated with respect to time. Temporal "alterations" or "modulations" can result from a comparison with conditions where release or delivery was "uncontrolled", or can mean that alterations or modulations are taking place during time. "Spatial" refers to any localised delivery.

An "active agent" in this context generally means a pharmacologically, therapeutically or otherwise effective agent which may have an effect itself or may be become active e.g. after being metabolised by endogenous enzymes or being converted under certain *in vivo* reaction conditions (e.g. pH).

Illustrative active agents that are useful in the present invention include the secretolytic agent ambroxol (an expectorant), drugs interfering with nerve transmitters, such as salbutamol (a sympathomimetic) and metoclopramide (a dopamine antagonist), the non-selective β-receptor blocking agent propranolol, the histamine receptor blocking agent ketotifen the bronchodilator theophylline. However, many other active agents selected from the groups of any agents orally applied and released into the gastrointestinal tract are also considered, such as anti-infective (e.g. anti-viral, anti-bacterial, fungicide) agents, non-steroidal anti-inflammatory agents (NSAIDs) and anti-inflammatory agents other than NSAIDs, lipid lowering agents, blood pressure affecting agents, immunosuppressants, anti-pyretic agents, analgetics, contraceptives, anti-cancer chemotherapeutics and others.

In general, active agents may be employed as free bases or any pharmaceutically acceptable salts thereof. Derivatives of active agents are also considered.

The term "basic active agent" as used herein refers to an active agent having an excess of basic functional groups compared to acidic functional groups. Examples of basic functional groups are amine and imine groups, but any other basic functional group known in the art is considered. Under acidic conditions, these agents are positively charged.

"Basic active agents" of low strength are those agents in which a filler excipient is added in order to increase the bulk size of the tablet for more convenient handling and manufacturing.

An "inactive matrix" in this context means those components of the controlled-release pharmaceutical composition which serve as a carrier for the active agent without being itself pharmacologically, therapeutically or otherwise effective. Preferably, the active agent becomes mixed with the inactive matrix components during preparation of the controlled-release pharmaceutical composition such that the active agent is dispersed or otherwise embedded within the inactive matrix.

Apart from chitosan, also other hydrophilic polysaccharide polymers belonging to the group of chitin, and salts and derivatives thereof, may be considered.

Apart from xanthan gum, further carbohydrate gums comprising tragacanth gum, gum karaya, guar gum, acacia gum and the like, and salts and derivatives thereof, may be considered.

When the term "% by weight" is used to indicate an amount, this refers to the composition's total weight, if not otherwise indicated.

Preferably, controlled-release pharmaceutical compositions of the present invention are intended for the preparation of tablets generated by compaction. However, use of the compositions for preparing other solid dosage forms, e.g. capsules, are also considered. Also comprised from the scope of the invention is any refinement of the solid dosage forms, e.g. coating of tablets.

In summary, the present invention provides a formulation of controlled-release pharmaceutical composition which is useful as universal composition for basic active agents. The composition comprise a binary mixture of hydrophilic polysaccharides: a combination of xanthan gum and chitosan (1:1) is particularly useful for controlled-release of basic active agents. Furthermore, a variation of the formulation for basic active agents is provided comprising pharmaceutically acceptable fillers which modified formulation is particularly useful for controlled-release of a basic active agent of low ionic strength.

In the present invention it is shown that the optimal binary polysaccharide mixture depends on the drug's solubility properties on the one hand and the kind of the drug, i.e. whether the drug is acidic or basic, on the other. For drugs having low water solubility, irrespective of being acidic or basic, the addition of a single polysaccharide, namely sodium alginate, was found to be sufficient to retard the drug release. Chitosan and xanthan gum combinations were advantageously suitable to control drug release of water-soluble basic drugs. For example, the optimum ratio of xanthan gum and chitosan was 1:1, when metoclopramide-HCl or propranolol-HCl were used as basic model drugs. In case of basic model drugs of low strength, there was the necessity for adding one or more fillers to increase the tablet size.

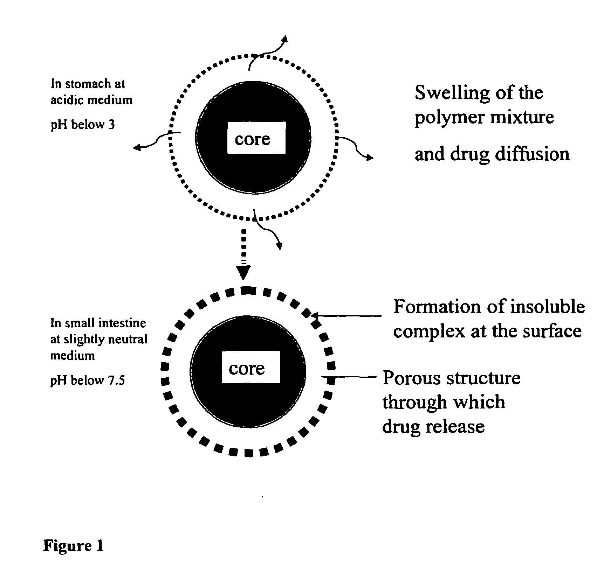

The combination of xanthan gum and chitosan (1:1) develops an insoluble hydrogel layer during the dissolution process. Occurring at the surface of a tablet, this reaction leads to the formation of an insoluble coat that is completed in the duodenal region. The drug is released then by diffusion. This mechanism is schematically illustrated in *Figure 1*.

### Detailed description of the invention

The invention shall now be further described and illustrated by the following examples with respect to the attached *Figures 1-9* and *Tables 1-3.* All examples are provided by way of example only, without any intended limitation to the scope of the invention.
**Figure 1** shows a schematic depiction of an oral dosage unit, e.g. a tablet, composed of a controlled-release pharmaceutical composition of the present invention.
   Passing the highly acidic stomach (pH<3), certain amounts of the pharmacologically active ingredient are released from the tablet. Upon reaching the small intestine and thus a more neutral, slightly alkaline medium (pH<7.5), an insoluble complex is formed *in situ* at the surface of the tablet followed by an extensive drug release.

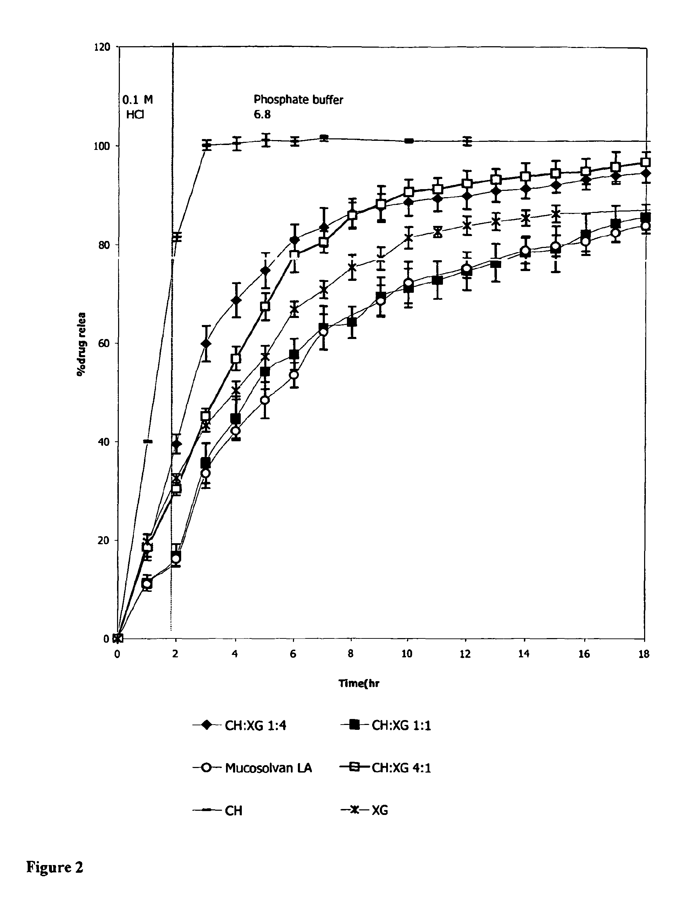

**Figure 2** shows the dissolution profiles of ambroxol-HCl (75 mg) from directly compressed matrix tablets. Each formulation was subjected to 0.1 M HCl for 2 hrs, then to phosphate buffer, pH 6.8. Dissolution conditions: RPM, 75; apparatus, USP basket; volume, 500 ml; temperature, 37°C. CH refers to chitosan, and XG refers to xanthan gum.

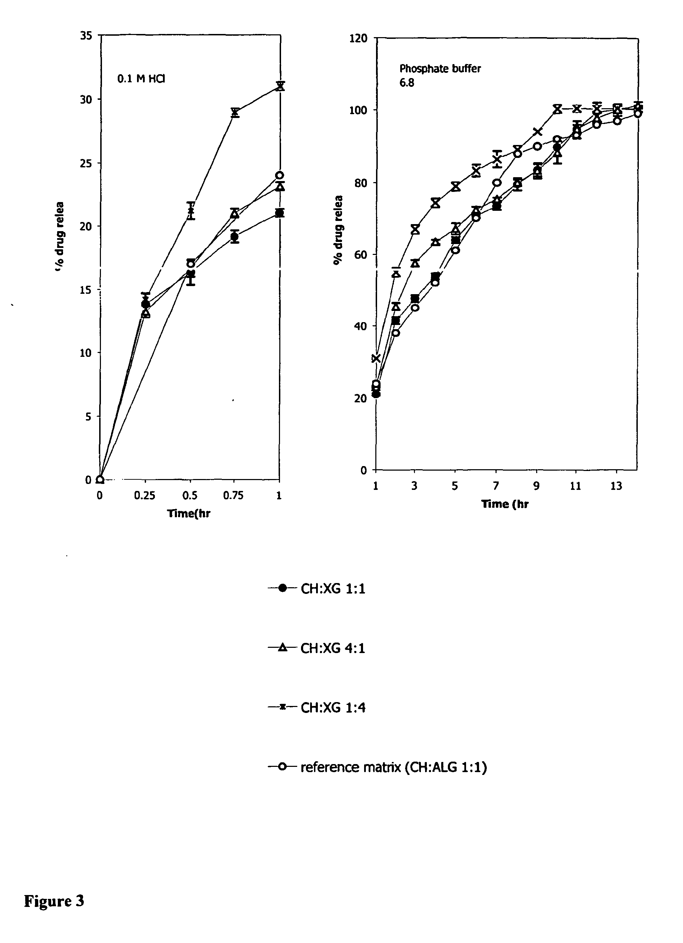

**Figure 3** shows the dissolution profiles of metoclopramide-HCl from matrix systems comprising xanthan gum and/or chitosan. Each formulation was subjected to 0.1 M HCl for 1 hr, then to phosphate buffer, pH 6.8. Dissolution conditions: RPM, 500; apparatus, USP paddle; volume, 600 ml; temperature, 37°C.

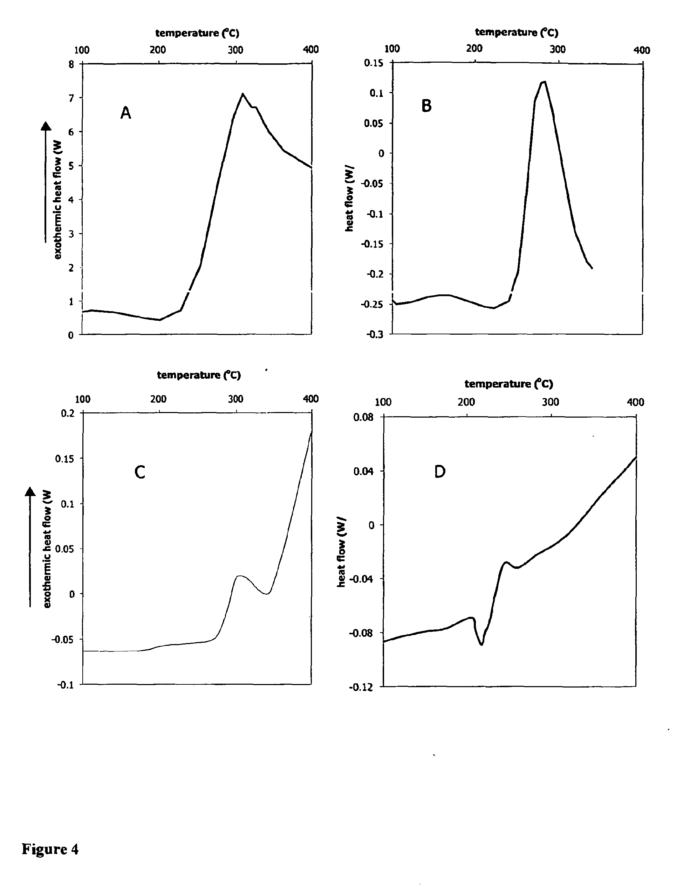

**Figure 4** shows the DSC thermograms of chitosan (A), xanthan gum (B), chitosan/xanthan gum (1:1) core material (C), and chitosan/xanthan gum (1:1) gel layer (D) obtained after an *in vitro* dissolution study, which includes the exposure of the matrices to 0.1 HCl for two hrs, and then the medium was replaced by phosphate buffer, pH 6.8, for the rest of 24 hrs. Thus, *Fig. 4* demonstrates that xanthan gum may bind to chitosan through ion pair interaction to form an insoluble complex (co-acervate).

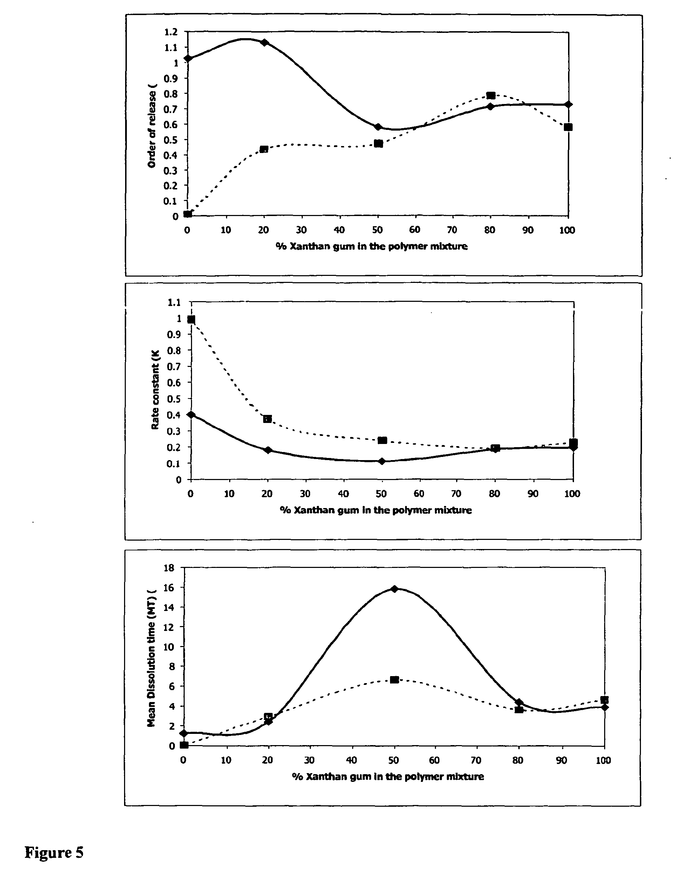

**Figure 5** shows the kinetic parameters of ambroxol-HCl released from the binary mixture of chitosan and xanthan gum. The drug to polymer ratio was 1:3. The continuous line represents the release parameters in acidic medium, and the dashed line represents the release parameters in neutral medium.

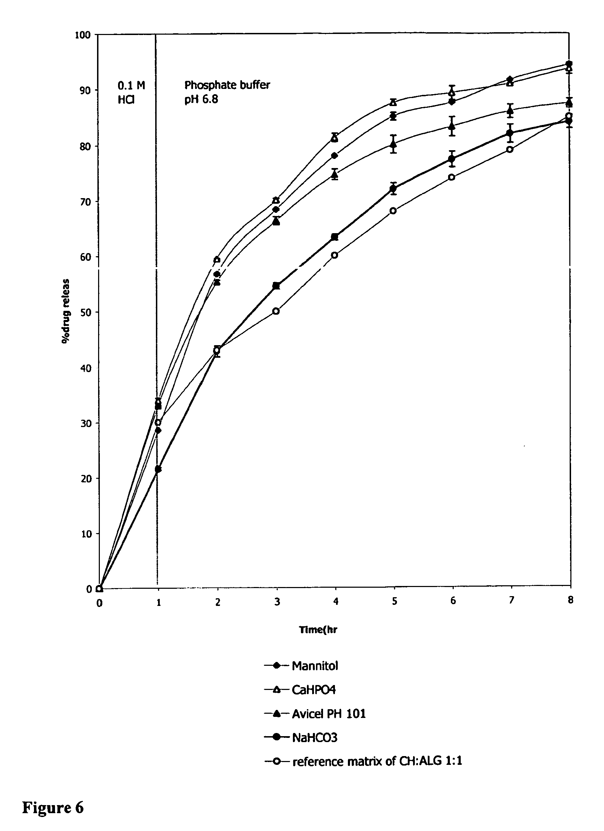

**Figure 6** shows the dissolution profile of salbutamol sulfate in different polysaccharide mixtures. Each formulation was subjected to 0.1 M HCl for 1 hr, then to phosphate buffer, pH 6.8. Dissolution conditions: RPM, 50; apparatus, USP paddle; volume, 500 ml; temperature, 37°C.

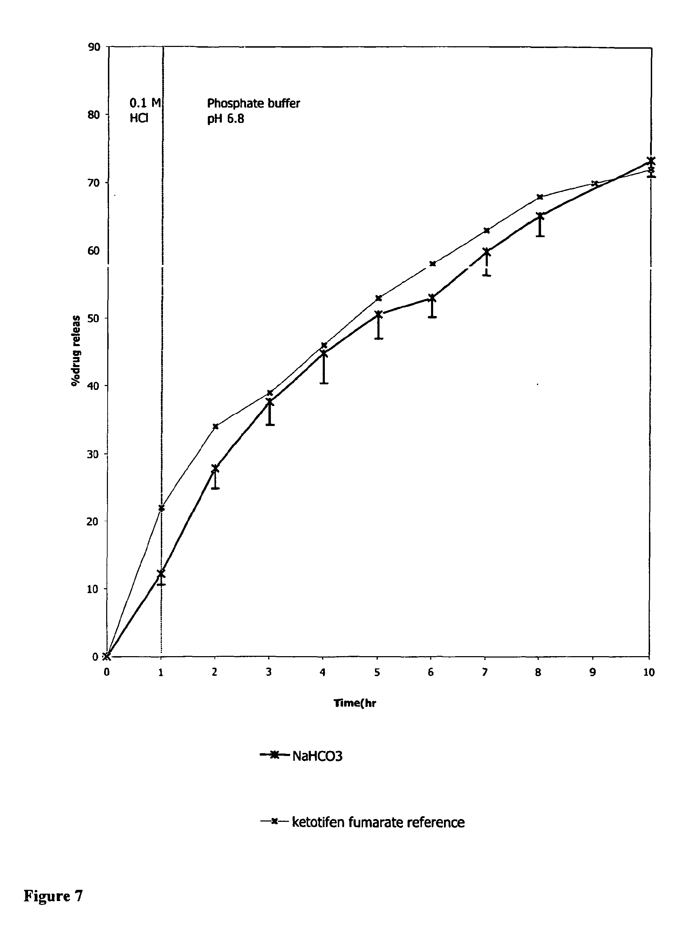

**Figure 7** shows the dissolution profile of ketotifen fumarate from different systems. Each formulation was subjected to 0.1 M HCl for 1 hr, then to phosphate buffer, pH 6.8. Dissolution conditions: RPM, 50; apparatus, USP paddle; volume, 500 ml; temperature, 37°C.

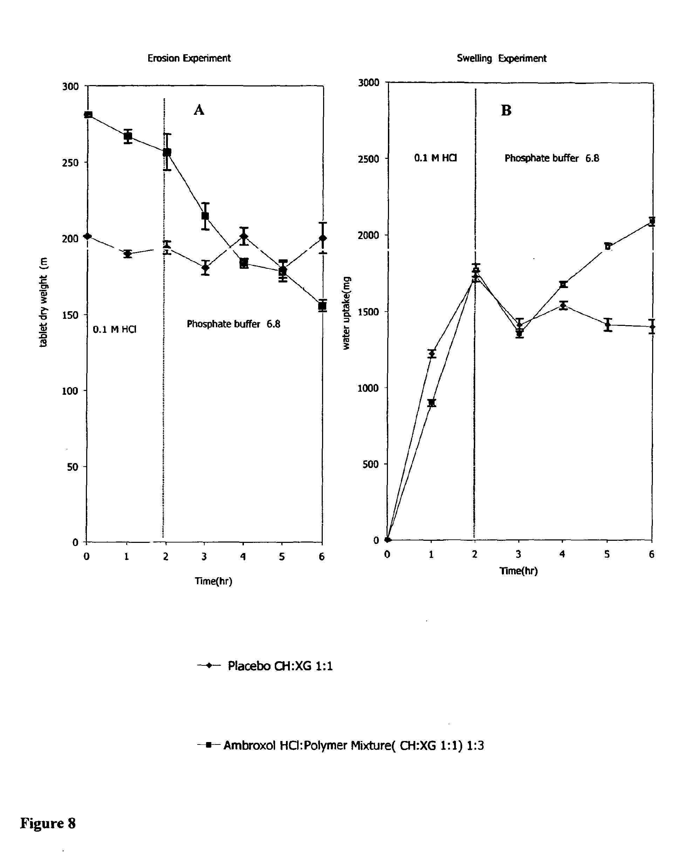

**Figure 8** shows the decrease in tablet dry weight (erosion) (A) and the water uptake (swelling) (B) during the dissolution process of chitosan/xanthan gum (1:1) matrices with ambroxol-HCl (drug loaded) and without ambroxol-HCl (drug unloaded).

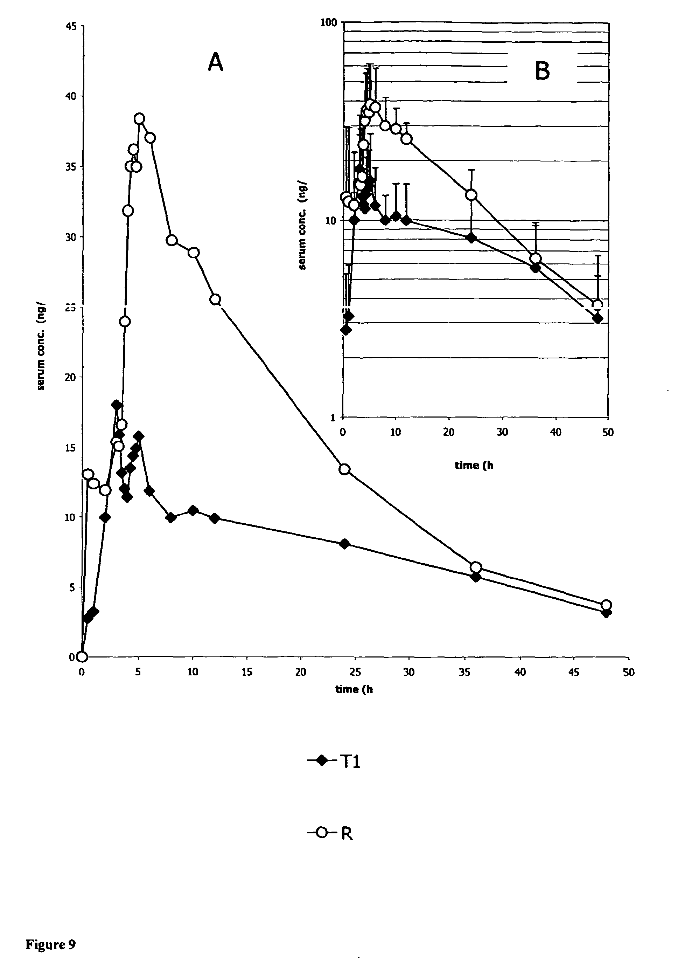

**Figure 9** shows an *in vivo* experiment comparing the kinetics of ambroxol-HCl serum concentrations after a single oral administration of 75 mg ambroxol-HCl via a controlled-release tablet of the present invention (referred to as T1) and Mucosolvan 75LA (referred to as R). Drug to polymer ratio of T1 was 1:3. The average of 6 subjects is shown in normal scale (A), and in semilog scale (B).

### Example 1: Preparation of controlled-release tablets and compaction studies

In order to assess the controlled-release properties and the matrix-forming ability of the hydrophilic polysaccharides which combination is object of the present invention, overall compaction studies, *in vitro* drug releasing assays and *in vivo* studies were conducted.

Pharmacologically active agents which were used as model basic agents in the present invention were ambroxol-HCl, metoclopramide-HCl, propranolol-HCl, salbutamol sulfate, ketotifen fumarate.

### 1. Preparation of controlled-release tablets

Polymers finally used were cationic chitosan and anionic xanthan gum (in a first approach, also anionic sodium alginate was tested). Drug to polymer ratio was set constant at 1:3 by weight (*cf*. *Table 1* below), irrespective of whether the polysaccharides were individually used or a binary polymer mixture was used instead. In the latter case, the ratios of chitosan to xanthan gum tested were 1:4, 1:1 and 4:1.

**Table 1: Summary of formulations used in the present invention**

| Model basic drug | Active ingredient per tablet [mg] | Tablet weight [mg] | D:P ratio | polymers and polymer ratio tested |
|---|---|---|---|---|
| Ambroxol-HCl (water-soluble) | 75 | 300 | 1:3* | CH, ALG, XG single CH:XG 1:1, 1:4, 4:1 |
| | | | | |
| Metoclopramide-HCl (water-soluble) | 30 | 120 | 1:3* | CH:XG 1:1, 1:4, 4:1 |
| | | | | |
| Propranolol-HCl (water-soluble) | 80 | 320 | 1:3* | CH:XG 1:1, 1:4, 4:1 |
| | | | | |
| Salbutamol sulfate (water-soluble, low strength) | 9.64⁺ | 80 | 1:3** | CH:XG 1:1 |
| | | | | |
| Ketotifen fumarate (water-soluble, low strength) | 2.75⁺⁺ | 80 | 1:3** | CH:XG 1:1 |
| | | | | |
| Theophyllin anhydrous (limited water-solubility) | 400 | 100 | 1:3* | CH, ALG, XG single |

| | | | | |
|---|---|---|---|---|
| CH, chitosan; ALG sodium alginate; XG, xanthan gum. + Eqivalent to 8 mg salbutamol ++ Equivalent to 2 mg ketotifen * Drug:polymer ratio ** (Drug/filler):polymer ratio | | | | |

Polysaccharide mixtures comprising the model basic agents were prepared in form of powders and granules (*cf*. below), and the mechanical properties were evaluated. The strength of tablets prepared from powder formulations was higher than that prepared from granules. However, no differences in drug release were observed.

The difference in tablet strength became obvious during compaction. Generally, granules possess better flow properties (are more flowable) than powder formulations but show larger elastic recovery stress upon compression. In the present invention, it was found that the ordinary granulation method resulted in the formation of porous granules. Such pores will contract air pockets upon compaction. These air pockets resulted in a decrease of tablet strength and in an increase in elastic recovery. Formation of small beads (granules) through modem technology may be the most appropriate solution. This would bring non-porous particles resulting in compaction with less elastic recovery and allowing easier handling by reducing the pressure applied in the tablet machine.

### 1.1. Tablet preparation from powder components

A model basic agent (*cf*. *Table 1*) was mixed with a single polysaccharide (xanthan gum or chitosan) and with a binary polysaccharide mixture of chitosan/xanthan gum, respectively. Prior to mixing, powders were deagglomerated by sieving. The components of each preparation were geometrically mixed by porcelain mortar and pestle for about 10 minutes before compression. Biplanar, cylindrical tablets were manufactured by compressing the powder mixtures applying a pressure of about 443 MPa for 15 seconds by a low speed compaction machine (hydraulic KBr press or Instron instrument). Tablets were sealed properly with aluminium foil and placed in amber glass bottles for 24 hours.

Since low strength drugs are water-soluble basic drugs as well, the optimal formula developed for those drugs was also applied to low strength drugs. However, a filler was added in order to increase the tablet size up to 20 mg. Different fillers (CaHPO₄, mannitol, Avicel pH101, and NaHCO₃) were tried by mixing a filler with an active agent at first, followed by the addition of a binary polysaccharide mixture. Similar to the high strength drugs, the ratio of low strengh drug/filler to polysaccharides was 1:3 (*cf. Table 1*).

The weight of the compressed mixture was the determinant in the die diameter: 13 mm for all formulations of high strength drugs to compress weights of 300-400 mg, and 6 mm for formulations of low strength drugs to compress weights of 80-120 mg.

### 1.2. Preparation of tablets from granules

The optimal formulation obtained with tablets prepared from powder components, namely chitosan/xanthan gum at a ratio of 1:1 and a drug to polymer ratio of 1:3, was used to prepare tablet starting material in granule form comprising ambroxol-HCl.

100 g powder mixture as described above *(cf. 1.1*.) was granulated with a 1:1 mixture of ethanol and water until a granulation wet mass was obtained (∼ 45 ml). The wet mass was forced through a sieve, mesh No. 20, and then dried at 65°C until a moisture content was detected similar to the corresponding powder formula using a Karl Fisher titrator. The dried granules were passed through a sieve, aperture size 500 µm. Samples were stored in well-closed bottles at room temperature. These granular mixtures were characterised before and after compression into tablet dosage forms. Biplanar, cylindrical tablets were manufactured by compressing the granules applying a pressure of about 443 MPa for 15 seconds by a hydraulic KBr press. The die diameter used was optimised to 13 mm to compress weights of 300-400mg.

### Example 2: Basic drugs

*In vitro* assays with basic drugs (and also with basic drugs of low strength, see below) were carried out by applying an acidic medium in the first two hours followed by a neutral medium for the rest of the experimental period (13).

### 1. Evaluation of optimal polymer ratios

In the development of controlled release formulations for basic drugs, ambroxol-HCl was chosen as a model for basic water-soluble molecules. In a first approach, ambroxol-HCl was added to xanthan gum and sodium alginate with a D:P ratio of 1:3. Ratios of xanthan gum and sodium alginate tested were 1:1, 1:4 and 4:1. There was no promising formulation that yielded a similar drug release pattern as Mucosolvan 75LA^{®} capsules, an ambroxol hydrochloride formulation commercially available from Boehringer Ingelheim (Germany). In addition to ambroxol hydrochloride, Mucosolvan 75LA capsules further comprise stearyl alcohol, carnauba wax, polyvinyl pyrrolidone, and magnesium stearate as excipients, and gelatin, titanium dioxide, yellow iron oxide, red iron oxide, erythrosin (E127), and indigotin (E132) as components of the capsule shell. Alternatively, chitosan was tested in a basic drug controlled-release composition. The suitable polymer mixture that results in a nearly similar drug release pattern as Mucosolvan 75LA was a mixture of chitosan and xanthan gum with a ratio of 1:1 (*Fig. 2*). This ratio showed the lowest drug release in both the acidic phase and the neutral phase among all combinations tested. Similar findings were also found with propranolol-HCl (*cf. Table 3* below) and metoclopramide (*Fig. 3*)*.*

**Table 2: Acceptance limits of extended release propranolol-HCl USP 24/NF 19 test 1 and the mean % drug release from different chitosan/xanthan gum mixtures at indicated time intervals.**

| time [hrs] | amount dissolved [%] | CH:XG 1: 1 | CH:XG 1:4 | CH:XG 4:1 | CH | XG |
|---|---|---|---|---|---|---|
| 1.5 | <30 | 23.08 (0.30) | 31.51 (0.38) | 30.14 (0.32) | 46.70 (2.11) | 31.06 (0.35) |
| 4 | 35-60 | 48.45 (1.00) | 56.88 (0.93) | 53.26 (0.65) | 71.57 (1.40) | 53.26 (0.81) |
| 8 | 55-80 | 61.66 (1.07) | 71.20 (0.97) | 66.09 (0.80) | 85.09 (2.03) | 69.21 (1.02) |
| 14 | 70-95 | 78.13 (1.80) | 85.63 (1.29) | 80.79 (1.43) | 93.60 (2.20) | 81.01(0.58) |
| 24 | 81-110 | 97.18 (2.41) | 103.69 (1.98) | 101.26 (0.52) | 111.00 (3.01) | 110.01 (1.14) |

| | | | | | | |
|---|---|---|---|---|---|---|
| Data in brackets represent S.D. Each formulation was subjected to pH 1.2 for the initial 1.5 hrs, followed by pH 6.8 for the rest of the dissolution process. CH, chitosan; XG, xanthan gum. | | | | | | |

### 2. Mechanism of controlled-release

The finding that xanthan gum, an anionic polymer, has the highest drug retardation power when mixed with chitosan in a ratio of 1:1 can be explained on the basis of complex formation. Xanthan gum may bind to chitosan to form an insoluble complex (co-acervate) through ion pair interaction *(**Fig. 4*). The complexation ratio was found to be 1:1 as proved by the DSC results (*cf*. *Table 3* below).

**Table 3: Measured melting temperature (Tₘ) and heat of fusion (ΔH_{f}) determined by DSC instrument of different gel layers of chitosan-xanthan gum mixtures developed after exposure to 0.1 M HCl for 2 hrs and to phosphate buffer pH 6.8.**

| Xanthan gum | Chitosan | Tₘ | | ΔH_{f} [J/g] | |
|---|---|---|---|---|---|
| | | average | SD | average | SD |
| 100 | 0 | 0 | 0 | 0 | 0 |
| 80 | 20 | 204.32 | 0.537 | 0.10 | 0.0353 |
| 50 | 50 | 210.60 | 3.154 | 3.97 | 0.325 |
| 20 | 80 | 223.57 | 1.393 | 2.01 | 0.132 |
| 0 | 100 | 0 | 0 | 0 | 0 |

The reaction between chitosan and xanthan gum might control drug permeation through the gel layer. The higher the degree of reaction between chitosan and xanthan gum, the more the retardation of the drug release would be. The reason might be that the two polymers develop a more restrict net work which renders drug diffusion. Consequently, the complexation ratio between chitosan and xanthan gum may occur at 1:1 ratio. This could be confirmed by the maximal decrease in drug diffusion through the matrix (lower drug release) among all combinations tested. The reaction between chitosan and xanthan gum in the gel layer might be completed in the neutral medium. The presence of an insoluble coat around the tablet and the absence of any dimensional increase in size were important signs for the completion of the reaction. Then, the drug release will be controlled by diffusion through this insoluble coat.

The releasing profiles of ambroxol-HCl (<60%) from different chitosan and xanthan gum ratios in acidic medium was fitted to Peppas equation (power equation) (19) using a nonlinear least square method. The rate of drug release was expressed in terms of *k* value, and the mechanism of release in terms of release exponent (*n*). In the acidic phase, the lowest *k* was obtained for a mixture of chitosan and xanthan gum with 1:1 ratio indicating the lowest rate of drug release. Also *n* indicates anomalous release but its value was almost the smallest and the closest one to 0.5. It is known that the reason for the deviations of the release profiles from square root of time kinetics (*n*=0.5) is the phenomenon of polymer relaxation, (20). This 1:1 ratio of chitosan/xanthan gum mixture involves the maximal contribution of drug with diffusion and the least polymer relaxation (least deviation from *n=*0.5) (*Fig. 5*).

Once the tablet comes into contact with neutral pH, e.g. reaching the small intestine, then the reaction between the positively charged chitosan and the negatively charged xanthan gum molecules is completed at the surface of the tablet. This leads to the formation of an insoluble porous coat from which the drug diffuses out to the dissolution medium.

The use of 10-20% NaHCO₃ as an excipient in the formulation leads to the following consequences: After placing the tablet in 0.1 M HCl, the tablet forms CO₂ bubbles around within 10 minutes. This gas will stick in at the surface because of the high viscosity of the gel layer formed. This will rise the tablet up to the surface of the dissolution medium. The presence of 10-20% NaHCO₃ has two functions. First, it will decrease the passage of the HCl molecules into the drug delivery system by making a gas (CO₂) around the tablet. This increases the tendency of drug release retardation. Second, a floating tablet will be formed. This leads to the delay in the gastric emptying rate of the tablet. In addition, this will decrease drug release since only the down face of the tablet is now facing the dissolution medium.

### 3. Swelling/erosion studies

The release of ambroxol-HCl from chitosan/xanthan gum (1:1) matrix was found to occur largely due to swelling mechanism than to erosion mechanism. This was confirmed by conduction a swelling/erosion study with drug-unloaded polymer matrix (chitosan/xanthan gum, 1:1), i.e. a placebo tablet, and with ambroxol-HCl-loaded matrix, i.e. a real tablet of the same polymer mixture with a drug to polymer ratio of 1:3 *(**Fig. 8*). The drug-unloaded matrix showed nearly no decrease in tablet weight during the *in vitro* dissolution experiment indicating insignificant erosion mechanisms in the polymer matrix compared to the swelling mechanism. While the drug-loaded matrix showed a decrease in tablet dry weight, of course, due to the release of the drug from the matrix (*Fig. 8A*), the rate of decrease in weight was almost equivalent to the rate of drug release from the dissolution profile. These findings indicated that the erosion mechanism has very little effect on the drug release from this matrix system. The study of water uptake is shown in *Fig. 8B* indicating the swelling kinetics of drug-loaded and drug-unloaded polymer matrices. The swelling effect is thought to be due to solvent penetration in the hydrophilic polymers within the matrix. At the beginning of the acidic phase, swelling occurred at a high rate, later, the rate was decreasing. This is due to the effect exerted by the swollen polymer layers at the surface that prevent solvent penetration to the inside of the gel layer. Swelling increases polymeric chain movement and the opportunity for the reaction. In the neutral medium, the reaction is completed between chitosan and xanthan gum. This might result in squeezing of water outside the developed water-insoluble complex co-acervate and forming a polymer-rich gel layer. This was manifested by the decrease in water uptake in the neutral medium during the first hour. The developed hydrogel would be less porous in the drug-unloaded matrix than the drug-loaded matrix. The release of drug out of the gel layer would result in the development of a larger porous structures. The pores facilitated more water penetration into the drug-loaded matrix compared to the drug-unloaded matrix. This explains the increase in water uptake ability of the drug-loaded matrix after two hours in the neutral medium.

### Example 3: Basic drugs of low strength

Salbutamol sulfate is a low strength water-soluble basic drug. The suggested formulation here would have a chitosan/xanthan gum ratio of 1:1, which previously has been developed for basic drugs (see above). The addition of a chitosan-xanthan gum mixture having a ratio of 1:1 with a D:P ratio of 1:3 was not sufficient to increase the tablet size. Low strength drugs always require the addition of a filler to increase the size of the tablet. Different fillers were tried in the formulation, namely CaHPO₄, mannitol, Avicel PH101 and NaHCO₃ (*Fig. 6*). The dissolution profile of the formulation containing NaHCO₃ was best among all formulations tested. When compared with a previous work, where chitosan and xanthan gum were used as drug-release retarding polymers, (21). The formulation of chitosan and xanthan gum with 1:1 ratio containing NaHCO₃ had the advantage of decreasing drug release in acidic medium *(**Fig. 6*). Another advantage of the addition of NaHCO₃ would be the development of a floating controlled-release drug delivery system. A floating system will increase the overall retention time in the gut, which may be attributed to the buoyancy of the tablet in the stomach (22).

Similar findings were observed with ketotifen fumarate, another low strength water-soluble basic drug *(**Fig. 7*). Also tested with the same matrix, i.e. chitosan-xanthan gum (1:1) matrix, and the filler was NaHCO₃. The release was compared to a previous work, where chitosan and sodium alginate were used as retarding polymers with a ratio of 1:1. The release of the drug from chitosan-xanthan (1:1) matrix using NaHCO₃ was slightly more retarded in acidic medium and nearly similar in basic medium (*Fig. 7*).

The feature disclosed in the foregoing description, in the claims and/or in the drawings may, both separately and in any combination thereof, be material for realising the invention in diverse forms thereof.

### Example 4: In vivo studies

A controlled-release tablet according to the present invention comprising 75 mg ambroxol-HCl and having a drug to polymer ratio of 1:3 was applied as an oral single dose to six healthy volunteers, and the serum concentrations were determined with time. Mucosolvan 75LA was used as a reference.

As demonstrated in *Fig. 9*, the controlled-release composition of the present invention resulted in a more constant release of ambroxol-HCl into the serum. In particular, the abrupt increase in serum concentration during the initial ten minutes produced by Mucosolvan 75LA was absent. Thus, the controlled-release composition of the present invention is superior to the reference formulation with regard to drug release retardation.

The features disclosed in the foregoing description, in the claims and/or in the drawings may, both separately and in any combination thereof, be material for realising the invention in diverse forms thereof.

### References

No.
1 V. Dhopeshwarkar and J. Zataz. Evaluation of Xanthan gum in the Preparation of Sustained Release Matrix Systems. Drug Dev. Ind. Pharm., 19, pp 999-1017, 1993.
2 M. Talukdar, R. Kinget. Swelling and Drug Release Behavior of Xanthan gum Matrix Tablet. Int. J. Pharm., 120, pp 63-72, 1995.
3 M. Talukdar., A. Michoel, P. Rombaut, R. Kinget. Comparative Study on Xanthan gum and HPMC as Matrices for Controlled Release Drug Delivery 1-Compaction and In Vitro drug release Behavior. Int. J. Pharm., 129, pp 233-241, 1996.
4 W. Paul, C. Sharma. Chitosan, a drug carrier for the 21st century: a review. STP Pharma Sci.,10, pp 5-22, 2000.
5 C. Lopez, A. Portero, M. Lemos, M. Nunez, P. Riveiro, J. Lopez, M. Alonso. Chitosan Microspheres for the Specific Delivery of Amoxycillin to the Gastric Cavity. S.T.P. Pharma. Sci. , 10, pp 69-76, 2000.
6 K. Oungbho;and B. Muller. Chitosan Sponges as Sustained Release Drug Carriers. Int.J. Pharm. 156, pp 229-237, 1997.
7 50. A. Bernkop-Schnurch, C. Paikl, C. Valenta. Novel Bioadhesion Chitosan-EDTA Conjugate Protects Leucine Enkeplalin from Degradation by Aminopeptidase N. Pharm. Res., 14, pp 917-922, 1997.
8 I. Orienti I, K. Aieda., E. Gianasi, C. Ponti, V. Zecchi. Chitosan Indomethacin Conjugates. Effect of Different Substituents on the Polysaccharide Molecule on Drug Release. Arch. Pharm. Pharm. Med, Chem. 329, pp 245-250, 1996.
9 F. Olivia, P. Buri., R. Gury. Chitosan: A unique Polysaccharide for Drug Delivery: a review. Drug Dev. Ind.Pharm. 24, pp 979-993, 1998.
10 L. Wan, L. Lim, B. Soh. Drug Release from Chitosan Beads. S.T.P. Pharma Sci, 4, pp 195-200, 1994.
11 C. Aral, J. Akbuga Alternative Approach to the preparation of Chitosan Beads. Int. J. Pharm., 168, pp 9-15, 1998.
12 Y. Murata, T. Maeda, Miyamoto, S. Kawashima. Preparation of Chitosan-reinforced alginate gel beads- effect of chitosan on gel matrix erosion. Int J Pharma. 96, pp 139-145, 1993.
13 A. Nokhodchi, Dj. Farid, M. Najafi, M. Adrangui. Studies on Controlled Release Formulations of Diclofenac Sodium. Drug Dev. Ind. Pharm., 23, pp1019-1023, 1997.
14 C.C. dos Santos Nogueira, L. Mendes Cabral, T.C. dos Santos. A. Marucci F. Alhaique, Evaluation of New Polysaccharides Networks for Extended-Release Purposes: Mesquite seed gum (MSG), Xanthan gum and Chitosan. Brazilian Journal of Pharmaceutical Sciences 39. 273-288,2003.
15 M.M. Meshali and K. E. Gabr. Effect of Interpolymer Complex Formation of Chitosan with Pectin or Acacia on the Release Behaviour of Chlororomazine HCl. Int. J. Pharm. 89. 177-181, 1993.
16 E.I. Hasan. B.I. Amro. T. Arafat. A.A. Badwan. Assessment of a Controller Release Hydrophilic Matrix Formulation for Metoclopramide HCl. European Journal of Pharmaceutics and Biopharmaceutics 55, 339-344, 2003
17 S. Dumitriu. N. Bruneau, H. Fessi. P. Buri. Polyionic Hydrogels as Support for Controlled Release of Nifedipine. Proceed. Int. 'I. Symp. Control. Rel. Bioact. Mater. 27. 455-456. 2000.
18 B.V. Murali Mohan Babu. K. Himasankar. Cheruvu P.S. Narayan, K.V. Ramana Murthy. Controlled Release of Diclofenac Sodium by Gum Karava-Chitosan Complex Coacervate: In Vivo Evaluation. Indian J. Pharm. Sci. 63. 408-412. 2001.
19 N. Peppas, P. Bures, Leobandung, Ichikawa. Hydrogelin Pharmaceutical Formulation. Eur. J. Pharm.Biopharm. 50, pp 2746,2000.
20 78. J. Mokel., B. Lippold. Zero-Order Drug Release from Hydrocolloid Matrices. Pharm. Res., 10, pp 1066-1070, 1993.
21 E. Hassan. Sustained Release Formulations Based on Chitosan and Alginate Mixtures (M.Sc. Thesis). Supervised by Dr. Bassam Amro and Dr. Adnan Badwan. Submitted to University of Jordan, Amman, Jordan. Dec., 1999.
22 127. S. Desai, S. Bolton. A Floating Controlled-Release Drug Delivery System: In Vitro In Vivo Evaluation. Pharm. Res., 10, pp 1321-1325, 1993.

## Claims

1. A controlled-release pharmaceutical composition comprising at least one basic active agent and an inactive matrix, said matrix comprising a mixture of hydrophilic polysaccharide polymers, said mixture consisting of chitosan and xanthan gum, wherein the ratio of xanthan gum and chitosan is about 1:1, and the ratio of basic active agent and hydrophilic polysaccharide polymer mixture is in the range from about 1:1 to about 1:5.

2. The controlled-release pharmaceutical composition according to claim 1, wherein the ratio of basic active agent and hydrophilic polysaccharide polymer mixture is about 1:3.

3. The controlled-release pharmaceutical composition according toclaim 1 or 2, wherein the basic active agent is selected from the group comprising ambroxol, metoclopramide, propranolol and salts and derivatives thereof.

4. The controlled-release pharmaceutical composition according to any of the preceding claims, optionally comprising one or more additional pharmaceutically acceptable fillers.

5. The controlled-release pharmaceutical composition according to claim 4, wherein the one or more pharmaceutically acceptable fillers are selected from the group comprising calcium hydrogen phosphate, mannitol, Avicel PH 101, sodium bicarbonate and the like.

6. The controlled-release pharmaceutical composition according to claim 4 or 5, wherein the ratio of basic active agent and the additional one or more pharmaceutically acceptable fillers on the one hand and hydrophilic polysaccharide mixture on the other hand is in the range of 1:1 to about 1:5.

7. The controlled-release pharmaceutical composition according to any of the claims 4 to 6, wherein the ratio of basic active agent and the additional one or more pharmaceutically acceptable fillers on the one hand and hydrophilic polysaccharide mixture on the other hand is about 1:3.

8. The controlled-release pharmaceutical composition according to any of claims 4 to 7, wherein the basic active agent is present in an amount from about 2.5% by weight to about 10% by weight, and wherein the one or more pharmaceutically acceptable fillers are present in an amount from about 15% by weight to about 22.5% by weight.

9. The controlled-release pharmaceutical composition according to any of the preceding claims, wherein the basic active agent is a basic active agent of low strength.

10. The controlled-release pharmaceutical composition according to claim 9, wherein the basic active agent of low ionic strength is selected from the group comprising salbutamol and ketotifen and salts and derivatives thereof.

11. A method for preparation of a controlled-release pharmaceutical composition according to any of the preceding claims, said method comprising the following steps:
(a) preparing a mixture of hydrophilic polysaccharide polymers consisting of chitosan and xanthan gum, wherein the ratio of xanthan gum and chitosan is about 1:1;
(b) mixing said mixture with a basic active agent, and optionally one or more additional pharmaceutically acceptable fillers;
(c) processing the preparation into granules;
(d) compacting the granules, preferably into a tablet.

12. Use of a controlled-release pharmaceutical composition according to any of claims 1 to 10 for the manufacture of a medicament for the treatment of an animal, preferably mammal, and most preferably man.

## Patentansprüche

1. Pharmazeutische Zusammensetzung zur gesteuerten Freisetzung, welche mindestens einen basischen Wirkstoff und eine inaktive Matrix umfasst, wobei die Matrix eine Mischung aus hydrophilen Polysaccharidpolymeren umfasst, wobei die Mischung aus Chitosan und Xanthangummi besteht, wobei das Verhältnis von Xanthangummi zu Chitosan ungefähr 1:1 beträgt, und wobei das Verhältnis von basischem Wirkstoff zur Mischung aus hydrophilen Polysaccharidpolymeren im Bereich von ungefähr 1:1 bis ungefähr 1:5 liegt.

2. Pharmazeutische Zusammensetzung zur gesteuerten Freisetzung nach Anspruch 1, wobei das Verhältnis von basischem Wirkstoff zur Mischung aus hydrophilen Polysaccharidpolymeren ungefähr 1:3 beträgt.

3. Pharmazeutische Zusammensetzung zur gesteuerten Freisetzung nach Anspruch 1 oder 2, wobei der basische Wirkstoff ausgewählt ist aus der Gruppe, umfassend Ambroxol, Metoclopramid, Propanolol und Salze und Derivate davon.

4. Pharmazeutische Zusammensetzung zur gesteuerten Freisetzung nach einem der vorangehenden Ansprüche, die wahlweise eine oder mehrere zusätzliche pharmazeutisch annehmbare Füllmittel umfasst.

5. Pharmazeutische Zusammensetzung zur gesteuerten Freisetzung nach Anspruch 4, wobei das eine oder die mehreren pharmazeutisch annehmbaren Füllmittel ausgewählt sind aus der Gruppe, umfassend Calciumhydrogenphosphat, Mannitol, Avicel PH 101, Natriumbicarbonat und Ähnliches.

6. Pharmazeutische Zusammensetzung zur gesteuerten Freisetzung nach Anspruch 4 oder 5, wobei das Verhältnis von basischem Wirkstoff und dem zusätzlichen einen oder den zusätzlichen mehreren pharmazeutisch annehmbaren Füllmitteln auf der einen Seite zur Mischung aus hydrophilen Polysacchariden auf der anderen Seite im Bereich von 1:1 bis ungefähr 1:5 liegt.

7. Pharmazeutische Zusammensetzung zur gesteuerten Freisetzung nach einem der Ansprüche 4 bis 6, wobei das Verhältnis von basischem Wirkstoff und dem zusätzlichen einen oder den zusätzlichen mehreren pharmazeutisch annehmbaren Füllmitteln auf der einen Seite zur Mischung aus hydrophilen Polysacchariden auf der anderen Seite ungefähr 1:3 beträgt.

8. Pharmazeutische Zusammensetzung zur gesteuerten Freisetzung nach einem der Ansprüche 4 bis 7, wobei der basische Wirkstoff in einer Menge von ungefähr 2,5 Gew.-% bis ungefähr 10 Gew.-% vorhanden ist, und wobei das eine oder die mehreren pharmazeutisch annehmbaren Füllmittel in einer Menge von ungefähr 15 Gew.-% bis ungefähr 22,5 Gew.-% vorhanden sind.

9. Pharmazeutische Zusammensetzung zur gesteuerten Freisetzung nach einem der vorangehenden Ansprüche, wobei der basische Wirkstoff ein basischer Wirkstoff von geringer (Ionen)Stärke ist.

10. Pharmazeutische Zusammensetzung zur gesteuerten Freisetzung nach Anspruch 9, wobei der basische Wirkstoff von geringer Ionenstärke ausgewählt ist aus der Gruppe, umfassend Salbutamol und Ketotifen und Salze und Derivate davon.

11. Verfahren zur Herstellung einer pharmazeutischen Zusammensetzung zur gesteuerten Freisetzung nach einem der vorangehenden Ansprüche, wobei das Verfahren die folgenden Schritte umfasst:
(a) Zubereiten einer Mischung aus hydrophilen Polysaccharidpolymeren, bestehend aus Chitosan und Xanthangummi, wobei das Verhältnis von Xanthangummi zu Chitosan ungefähr 1:1 beträgt;
(b) Mischen der Mischung mit einem basischen Wirkstoff und wahlweise einem oder mehreren zusätzlichen pharmazeutisch annehmbaren Füllmitteln;
(c) Verarbeiten der Zubereitung zu Granulat;
(d) Kompaktieren des Granulats, vorzugsweise zu einer Tablette.

12. Verwendung einer pharmazeutischen Zusammensetzung zur gesteuerten Freisetzung nach einem der Ansprüche 1 bis 10 zur Herstellung eines Medikaments zur Behandlung eines Tiers, vorzugsweise eines Säugetiers, und am stärksten bevorzugt eines Menschen.

## Revendications

1. Composition pharmaceutique à libération contrôlée comprenant au moins un agent actif basique et une matrice inactive, ladite matrice comprenant un mélange de polymères de polysaccharides hydrophiles, ledit mélange étant constitué de chitosane et d'une gomme de xanthane, où le rapport de la gomme de xanthane et du chitosane est d'environ 1:1, et le rapport de l'agent actif basique et du mélange de polymères de polysaccharides hydrophiles est dans la plage d'environ 1:1 à environ 1:5.

2. Composition pharmaceutique à libération contrôlée selon la revendication 1, dans laquelle le rapport de l'agent actif basique et du mélange de polymères de polysaccharides hydrophiles est d'environ 1:3.

3. Composition pharmaceutique à libération contrôlée selon la revendication 1 ou 2, dans laquelle l'agent actif basique est sélectionné parmi le groupe comprenant l'ambroxol, le métoclopramide, le propranolol et leurs sels et leurs dérivés.

4. Composition pharmaceutique à libération contrôlée selon l'une quelconque des revendications précédentes, comprenant optionnellement un ou plusieurs agents de charge supplémentaires pharmaceutiquement acceptables.

5. Composition pharmaceutique à libération contrôlée selon la revendication 4, dans laquelle les un ou plusieurs agents de charge pharmaceutiquement acceptables sont sélectionnés parmi le groupe comprenant l'hydrogénophosphate de calcium, le mannitol, l'Avicel PH 101, le bicarbonate de sodium et autres.

6. Composition pharmaceutique à libération contrôlée selon la revendication 4 ou 5, dans laquelle le rapport de l'agent actif basique et des un ou plusieurs agents de charge pharmaceutiquement acceptables supplémentaires d'une part et du mélange de polysaccharides hydrophiles d'autre part est dans la plage de 1:1 à environ 1:5.

7. Composition pharmaceutique à libération contrôlée selon l'une quelconque des revendications 4 à 6, dans laquelle le rapport de l'agent actif basique et des un ou plusieurs agents de charge pharmaceutiquement acceptables supplémentaires d'une part et du mélange de polysaccharides hydrophiles d'autre part est d'environ 1:3.

8. Composition pharmaceutique à libération contrôlée selon l'une quelconque des revendications 4 à 7, dans laquelle l'agent actif basique est présent dans une proportion d'environ 2,5 % en poids à environ 10 % en poids, et dans laquelle les un ou plusieurs agents de charge pharmaceutiquement acceptables sont présents dans une proportion d'environ 15 % en poids à environ 22,5 % en poids.

9. Composition pharmaceutique à libération contrôlée selon l'une quelconque des revendications précédentes, dans laquelle l'agent actif basique est un agent actif basique de faible force.

10. Composition pharmaceutique à libération contrôlée selon la revendication 9, dans laquelle l'agent actif basique de faible force ionique est sélectionné parmi le groupe comprenant le salbutamol et le kétotifène et leurs sels et dérivés.

11. Procédé de préparation d'une composition pharmaceutique à libération contrôlée selon l'une quelconque des revendications précédentes, ledit procédé comprenant les étapes suivantes :
(a) préparation d'un mélange de polymères de polysaccharides hydrophiles constitués de chitosane et de gomme de xanthane, où le rapport de la gomme de xanthane et du chitosane est d'environ 1:1,
(b) mélange dudit mélange avec un agent actif basique, et optionnellement avec un ou plusieurs agents de charge pharmaceutiquement acceptables supplémentaires,
(c) élaboration de la préparation en granules,
(d) compactage des granules, de préférence en un comprimé.

12. Utilisation d'une composition pharmaceutique à libération contrôlée selon l'une quelconque des revendications 1 à 10 pour la fabrication d'un médicament pour le traitement d'un animal, de préférence un mammifère et de la façon la plus préférée un être humain.
